# EUROPÄISCHE PATENTSCHRIFT

(11) **EP 0 704 536 B1**
(45) Veröffentlichungstag und Bekanntmachung des Hinweises auf die Patenterteilung: **18.11.1998**
(21) Anmeldenummer: 95113496.4
(22) Anmeldetag: 28.08.1995
(51) Int. Cl.: C12P 19/30

(54) **Verfahren zur Isolierung und Reinigung von nukleotidaktivierten Zuckern aus biologischen Quellen**
Method for the isolation and purification of nucleotid-activated sugars from biological sources
Méthode par l'isolement et la purification de sucres, activés par des nucléotides, provenants de sources biologiques

(30) Priorität: 02.09.1994 DE 4431280
(43) Veröffentlichungstag der Anmeldung: 03.04.1996
(73) Patentinhaber: HOECHST AKTIENGESELLSCHAFT, 65929 Frankfurt am Main (DE)
(72) Erfinder: Seiffert-Störiko, Andreas, Dr., D-65929 Frankfurt (DE); Hörsch, Brigitte, Dr., D-65830 Kriftel (DE); Marquardt, Rüdiger, Dr., D-60389 Frankfurt (DE); Kretzschmar, Gerhard, Dr., D-65760 Eschborn (DE); Meiwes, Johannes, Dr., D-65510 Idstein (DE)

(56) Entgegenhaltungen:
- EP-A- 0 524 143
- JOURNAL OF BIOLOGICAL CHEMISTRY, Bd. 260, Nr. 15, 25.Juli 1985 MD US, Seiten 8838-8849, HERMAN H. HIGA ET AL. 'Sialylation of glycoprotein oligosaccharides with N-Acetyl-, N-Glycoyl-, and N-O-Diacetylneuraminic acids'
- TETRAHEDRON LETTERS, Bd. 29, Nr. 7, 1988 OXFORD GB, Seiten 789-790, CLAUDINE AUGE ET AL. 'An efficient synthesis of Cytidine Monophospho-Sialic acids with four immobilized enzymes'

## Beschreibung

Die vorliegende Erfindung betrifft ein verbessertes Verfahren zur Isolierung und Reinigung von Zuckernukleotiden, insbesondere von Cytidin-Monophosphataktivierter N-Acetyl-Neuraminsäure (CMP-Nana), aus biologischen Quellen.

Zuckermoleküle finden sich in allen Zellen und haben eine große Bedeutung für die Lebensvorgänge. Sie dienen nicht nur als Nahrungsgrundlage, sondern haben auch große Bedeutung als Stützfunktion (Zellwände) und in den Nukleinsäuren. Zucker sind als Monomere, Oligomere und Polymere in der Natur vorhanden.

Damit Zuckermoleküle intrazellulär mit anderen Molekülen chemisch verbunden bzw. polymerisiert werden können, müssen sie vorher aktiviert werden. Diese Aktivierung erfolgt entweder über eine Anlagerung von Phosphat oder über die Derivatisierung mit Nukleotiden. Beide Reaktionen werden von speziellen Enzymen (Kinasen bzw. Glycosyltransferasen) katalysiert und sind häufig nacheinander geschaltet. In biologischen Zellen sind die einzelnen Zucker meist mit einem bestimmten Nukleosid-Diphosphat verknüpft, das die Aktivierung bewirkt. Im Falle der N-Acetyl-Neuraminsäure ist es beispielsweise das Cytidin, welches jedoch als 5'-Monophosphat, d. h. als Cytidin-Monophosphat-N-Acetyl-Neuraminsäure vorliegt.

Bei Bakterien (z. B. Neisserien, Streptococcen) ist N-Acetyl-Neuraminsäure (Nana), neben weiteren Zuckern, als Polymerbestandteil (Heteropolymer) auf Zelloberflächen vorhanden. Dabei liegt Nana in verschiedenen Verknüpfungen vor; bei E. coli z. B. als extrazelluläres Homopolymer, der Colominsäure. Hier findet sich jedoch stets die 2,8- und 2,9-Verknüpfung (Reglero et al., Int. J. Biochem., Vol. 25, No. 11, p. 1517-1527, 1993).

Die Biosynthese dieser Zellwandbestandteile geschieht intrazellulär. Zunächst wird Nana mit Hilfe des Enzyms CMP-Nana-Synthase (EC 2.7.7.43) unter Verbrauch von Cytidin-Triphosphat (CTP) zu CMP-Nana aktiviert (Kean, Glycobiol., Vol. 1, No. 5, p. 441-447, 1991) und anschließend mittels Sialyltransferasen zu Polymer-Untereinheiten verbunden. Diese Polymer-Untereinheiten werden mit Hilfe eines Lipid-Carriers aus den Zellen geschleust und extrazellulär zu einem Molekül, dem eigentlichen Polymer, verknüpft (Shockman und Barrett, Ann. Rev. Microbiol., Vol. 37, p. 501-527, 1983).

Auch in eukaryontischen Zellen findet sich N-Acetyl-Neuraminsäure, die außer auf der Zellwand auch häufig als endständiges Molekül bei Zuckerketten an Glykoproteinen vorkommt. Diese Zuckerketten dienen der Erkennung der Zellen untereinander oder sind für die Strukturerhaltung der Proteine notwendig.

Die Bedeutung der Oligosaccharide in biologischen Prozessen wird zunehmend als Möglichkeit für therapeutische Eingriffe angesehen. So wären Pharmazeutika (z. B. entzündungshemmende Wirkstoffe) auf Oligosaccharid-Basis denkbar (WO 91/19502, WO 92/22661, WO 92/22565, WO 92/22563, europ. Offenlegungsschriften 0089 938, 0089 939, 0089 940). Problematisch ist hierbei jedoch die Herstellung ausreichender Mengen an Oligosacchariden, die aus spezifisch miteinander verknüpften Monosacchariden aufgebaut sind. Durch die hohe Anzahl funktioneller Gruppen sind von einem Oligosaccharid viele verschiedene Isomere und verschiedene Verknüpfungsmöglichkeiten vorstellbar, von denen lediglich eines die gewünschte biologische Aktivität aufweist. Eine ausschließlich chemische Synthese von Oligosacchariden mit der gewünschten Konfiguration verlangt den Einsatz von Schutzgruppen, ist umständlich und kostenintensiv.

Ein Einsatz von Enzymen zur Herstellung dieser biologisch aktiven Oligosaccharide wäre dazu eine wünschenswerte Alternative. Diese Enzyme (Glycosyltransferasen) sind in biologischem Material ubiquitär vorhanden und können daraus isoliert werden (z. B. Beyer et al., Adv. Enzymol., Vol. 52, p. 23-175, 1981). Sie besitzen eine hohe Spezifität in Bezug auf das Substrat und den Akzeptor und somit auf die Art der chemischen Verknüpfung und erlauben teilweise sogar den Einsatz von Derivaten.

Bei der Verwendung dieser Glycosyltransferasen ist die Anwesenheit von Zuckernukleotiden als Donor-Substrate erforderlich. Diese Zuckernukleotide sind teuer, chemisch labil und aufwendig zu isolieren. Eine Alternative zur Herstellung der Zuckernukleotide mit Enzymen ist die chemische Totalsynthese, die jedoch ebenfalls sehr aufwendig und teuer ist.

Eine direkte Isolierung der Zuckernukleotide aus biologischem Material ist denkbar. Diese Moleküle liegen jedoch intrazellulär in sehr geringer Konzentration vor, sodaß sich eine Aufarbeitung nur in bestimmten Fällen lohnt. Bei aufwendigen Aufarbeitungsschritten kommt es häufig zu einer spontanen Zersetzung der Moleküle und damit zu einer geringeren Ausbeute.

Daher ist ein verbessertes, zeitsparendes Verfahren zur Isolierung von Zuckernukleotiden direkt aus biologischen Quellen ohne die vorangehende Isolierung der Enzyme, die Zuckernukleotide synthetisieren, wünschenswert.

Bereits 1959 wurde CMP-Nana im E. coli Stamm K-235 (ATCC 13027) detektiert und aus den Zellen isoliert (Comb et al., J. Am. Chem. Soc., Vol. 81, p. 5513-5514). Die Zellen wurden dazu mit Ultraschall aufgeschlossen und das CMP-Nana mit Hilfe eines Anionenaustauschers gereinigt (Dowex-1, Cl⁻; Elution mit LiCl). In einer weiteren Arbeit (Comb et al., J. Biol. Chem., Vol. 241, Nr. 23, p. 5637-5642, 1966) wird zur Isolierung von CMP-Nana vorgeschlagen, nach Lyse der Zellen mit verschiedenen Methoden (Ultraschall, French press etc.) zunächst Nukleotide und Proteine mit Ethanol zu fällen. Dazu werden die Zellen zunächst in Gegenwart von Aceton getrocknet, mit 80 % Ethanol versetzt, bei Raumtemperatur für 12 Stunden stehen gelassen und abzentrifugiert. Die Nukleotide in dem Extrakt werden an einen Ionenaustauscher (Dowex-1, HCO₃-, 200-400 mesh) gebunden und mit Triethylammonium-Hydrogencarbonat bei pH 7,4 eluiert. Der Anteil an CMP-Nana an der Nukleotid-Gesamtmenge beträgt je nach Qualität der Zellen 1 bis 10 %. Bei der Aufarbeitung treten jedoch Probleme auf. So muß der Ionenaustauscher über mehrere Stunden mit 80 %igem ethanolischem Puffer äquilibriert werden, und während der Elution kommt es zu einer Verringerung der Fließgeschwindigkeit des Puffers in der Säule (Zeitfaktor).
Die CMP-Nana-haltigen Fraktionen werden schließlich vereinigt und mittels Papierchromatographie oder mittels Chromatographie an Sephadex® G-25 weiter gereinigt.

Bei den beschriebenen Verfahren liegt der Nachteil insbesondere darin, daß gemessen am potentiellen Bedarf an CMP-Nana lediglich geringe Mengen an CMP-Nana mit großem Aufwand, zeitlich unökonomisch und schwer reproduzierbar bereitgestellt werden können.

Für alle übrigen Zuckernukleotide ist eine Isolierung mit den oben angegebenen Methoden denkbar.

Neben der Möglichkeit Zuckernukleotide aus Zellextrakten zu isolieren, ist man teilweise dazu übergangen, die Enzyme für die Herstellung der aktivierten Zucker zu isolieren und für eine Synthese einzusetzen. Für diese Herstellprozesse finden sich vielfältige Beispiele in der Literatur. Nukleotid-aktivierte Zucker sind z. B. UDP-Glukose, UDP-Galaktose, UDP-N-Acetyl-Glukosamin, UDP-N-Acetylgalaktosamin, UDP-Glukuronsäure, GDP-Fucose, GDP-Mannose, dTDP-Glukose, dTDP-Galaktose und CMP-N-Acetyl-Neuraminsäure.

Nachfolgend sind einige Arbeiten zitiert, die sich mit der enzymatischen Herstellung von CMP-Nana und deren Aufarbeitung beschäftigen. So synthetisierten Shames et al. (Glycobiol., Vol. 1, No. 2, p. 187-191, 1991) mit Hilfe der in einen E. coli-Überexpressionsvektor klonierten CMP-Nana-Synthase CMP-Nana und einige Derivate, die durch Fällung mittels Ethanol und anschließender Trocknung isoliert wurden. Liu et al. (J. Am. Chem. Soc., Vol. 114, p. 3901-3910, 1992) reinigten CMP-Nana aus einem enzymatischen Reaktionsansatz zur Abtrennung der Nukleotide durch Chromatographie an einem Anionenaustauscher (Dowex-1, Formiat-Form) und anschließend zur Abtrennung des überschüssigen Ammonium-Bicarbonates durch Chromatographie an einem Kationenaustauscher (Dowex 50W-X8, H⁺-Form). Andere Autoren stellen CMP-Nana ebenfalls enzymatisch her und reinigen das Produkt mit Hilfe der präparativen HPLC (Gross et al., Joint Meeting, Basel, Vol. 366, p. 795-796, 1985), durch Bindung an Aktivkohle und nachfolgender Elution mit 0,1M NH₄Cl in 50 % Ethanol (Shoyab et al., J. Neurochem., Vol. 11, p. 639-646, 1964), durch Reinigung mittels Papierchromatographie auf Whatman 3MM-Blättern (van den Eijnden und Dijk, Hoppe-Seyler's Z. Physiol. Chem., Bd. 353, p. 1817-1820, 1972), oder mit Hilfe einer Kieselgel-Säulenchromatographie (Augé und Gautheron, Tetrahedron Lett., Vol. 29, Nr. 7, p. 789-790, 1988). Diese und andere Autoren reinigten CMP-Nana mit einem Propanol:Wasser- (7:3) Gemisch, bzw. mit einem Ethanol:Ammonium-Acetat pH 6,5- (7:3) Laufmittelsystem (Higa und Paulson, J. Biol. Chem., Vol. 260, p. 8838-8849, 1985).

Bei allen übrigen Zuckernukleotiden sind vergleichbare Reinigungsprozesse bekannt und beschrieben. Dem Fachmann bekannte Verfahren zur enzymatischen Herstellung von Nukleotid-aktivierten Zuckern sind z. B.: Kittelmann et al., Annals New York Acad. Sci., Vol. 672, Enzyme Engineering, p. 444-450, 1992; Makino et al., Tetrahedr. Lett., Vol. 34, p. 2775, 1993; Martin et al., Vol. 34, p. 1765, 1993; Europ. Patentanmeldung 0524 143 A1; Ikeda, Carbohydr. Res., Vol. 242, p. 123, 1992; Kean, Glycobiol., Vol. 1, p. 441, 1991; Ichikawa et al., J. Org. Chem., Vol. 57, p. 2943, 1993; Adelhorst et al., Carbohydr. Res., Vol. 242, p. 69, 1993; Schmidt et al., Lieb. Ann. Chem., p.121, 1991; Stiller et al., Lieb. Ann. Chem., p. 467, 1992; Heidlas et al., J. Org. Chem., Vol. 57, p. 146, 1992; Heidlas, Acc. Chem. Res., Vol. 25, p. 307, 1992; Simon et al., J. Org. Chem., Vol. 55, p. 1834, 1990; Wong et al., J. Org. Chem., Vol. 57, p. 4343, 1992; Pallanca et al., J. Chem. Soc. Perkin Trans. 1, p. 3017, 1993.

Aufgrund der oben erwähnten Möglichkeiten der Erforschung des Potentials an Pharmazeutika auf Oligosaccharid-Basis und deren Aufbau über enzymatische Glykosylierungsreaktionen besteht daher ein Bedürfnis nach einem Verfahren zur Herstellung von Zuckernukleotiden in beliebiger Menge und in gleichbleibender Qualität.

Aufgabe der vorliegenden Erfindung ist es, ein verbessertes Verfahren zur Isolierung von Zuckernukleotiden aus biologischen Quellen (Extrakten oder enzymatischen Ansätzen) bereitzustellen, das die eingangs erwähnten Nachteile des Standes der Technik nicht aufweist.

Die gestellte Aufgabe wird gelöst durch ein Verfahren zur Isolierung und Reinigung von Zuckernukleotiden aus biologischen Quellen, bei welchem eine notwendigenfalls von unlöslichen Zellbestandteilen befreite, zuckernukleotidhaltige Lösung, die ein Zellextrakt oder eine Reaktionslösung eines enzymatischen Reaktionsansatzes sein kann, nach Entfernung gelöster Proteine mittels alkoholischer Proteinfällung bis zur Trockne eingeengt, der resultierende, zuckernukleotidhaltige Rückstand in einem Laufmittelgemisch aufgenommen und an Kieselgel chromatographiert wird, dadurch gekennzeichnet, daß der zuckernukleotidhaltige Rückstand in einem aus einer Mischung eines kurzkettigen Alkohols mit einer 0,5 bis 1 molaren wäßrigen Lösung eines Ammoniumsalzes in einem Volumenverhältnis von 1:1 bis 1:10 bestehenden Laufmittelgemisch aufgenommen, mit trockenem Kieselgel vermengt als zähe Masse von honigartiger Konsistenz auf eine chromatographische Säule mit handelsüblichem Kieselgel beliebiger Korngröße als stationärer Phase aufgetragen und mit dem genannten Laufmittelgemisch unter Druck eluiert wird.

Vorzugsweise besteht das Laufmittelgemisch aus einer Mischung eines kurzkettigen Alkohols mit einer 0,5 bis 1 molaren wäßrigen Lösung eines Ammoniumsalzes in einem Volumenverhältnis von 1:1 bis 1:2, insbesondere in einem Volumenverhältnis von 1:1,25.

Die wäßrige Lösung des Ammoniumsalzes ist vorzugsweise 1 molar.

Vorzugsweise ist im Laufmittelgemisch der kurzkettige Alkohol 2-Propanol, das Ammoniumsalz Triethylammoniumhydrogencarbonat.

Das erfindungsgemäße Verfahren eignet sich besonders gut zur Isolierung und Reinigung von Cytidin-Monophosphat-N-Acetyl-Neuraminsäure (CMP-Nana).

Das erfindungsgemäße Verfahren eignet sich jedoch auch zur Isolierung und Reinigung sämtlicher übrigen nukleotidaktivierten Zucker und deren Derivate aus biologischen Quellen (Zellextrakte und enzymatische Ansätze), insbesondere zur Isolierung und Reinigung von Derivaten von CMP-Nana, die aus enzymatischen Ansätzen gewonnen wurden.
Als Derivate der N-Acetyl-Neuraminsäure seien hier genannt:
N-Acetyl-4-O-Acetyl-Neuraminsäure (Neu4,5Ac₂), N-Acetyl-9-O-Acetyl-Neuraminsäure (Neu5,9Ac₂), N-Acetyl-7,9-di-O-Acetyl-Neuraminsäure (Neu5,7,9Ac₃), N-Acetyl-9-O-Lactoyl-Neuraminsäure (Neu4Ac9Lt), N-Acetyl-4-O-Acetyl-9-O-Lactoyl-Neuraminsäure (Neu4,5Ac₂9Lt), N-Acetyl-Neuraminsäure-9-Phosphat (Neu5Ac9P), N-Glycolyl-Neuraminsäure (Neu5Gc), N-Glycolyl-9-O-Acetyl-Neuraminsäure (Neu9Ac5Gc), N-Glycolyl-9-O-Lactoyl-Neuraminsäure (Neu5Gc9Lt), N-Glycolyl-Neuraminsäure-8-Sulfat (Neu5Gc8S). Außerdem kommen noch dazu: 5-Azido-Neuraminsäure, N-Acetyl-9-Azido-9-Deoxy-Neuraminsäure, N-Acetyl-9-Acetamido-9-Deoxy-Neuraminsäure, Carbomethoxy-N-Acetyl-Neuraminsäure und Carbobenzyloxy-N-Acetyl-Neuraminsäure.

Nachfolgend wird das Verfahren gemäß der vorliegenden Erfindung ausgehend von einer Zuckernukleotid-haltigen Lösung (Zellextrakt oder enzymatischer Ansatz) detailliert beschrieben.

Die Zuckernukleotid-haltige Lösung wird mit Alkohol, z. B. Ethanol oder Propanol bis zu einer Endkonzentration von 40 bis 60 %, insbesondere etwa 50 % (Vol./Vol.) gemischt und eine Stunde bei 4°C inkubiert. Die ausgefallenen Proteine, sowie die unlöslichen Zellbestandteile und, falls verwendet, Glasperlen, werden durch geeignete Weise abgetrennt und der verbliebene Überstand im Vakuum eingeengt (einrotiert bzw. lyophilisiert).

Der zur Trockne eingeengte Überstand wird in dem Laufmittel für die anschließende Kieselgel-Säulenchromatographie gelöst und mit trockenem Kieselgel vermischt, so daß eine zähe Masse von honigähnlicher Konsistenz entsteht. Als Laufmittel eignet sich eine Mischung aus kurzkettigen Alkoholen und 0,5 bis 1 molaren wäßrigen Lösungen von Ammoniumsalzen im Volumenverhältnis von 1:1 bis 1:10, vorzugsweise 1:1 bis 1:2. Besonders eignet sich als Laufmittel ein Gemisch aus 2-Propanol und einer 1M wäßrigen Lösung von Triethylammoniumhydrogencarbonat im Volumenverhältnis von 1:1,25. Handelsübliche Kieselgele beliebiger Korngrößen können für die Trennung eingesetzt werden. Die aufgetragene Mischung wird unter Druck eluiert.

Die aufgefangenen Fraktionen werden mittels geeigneter Detektionsverfahren untersucht, bevorzugt mit Hilfe der Dünnschicht-Chromatographie (DC) oder der HPLC. Die DC kann auf einem geeigneten Träger (z. B. Kieselgel-60 HPTLC-Platten o. ä.) durchgeführt werden. Dabei bietet sich als Laufmittel das oben erwähnte Elutionsmittel an. Für die Analytik mit Hilfe der HPLC bieten sich die Literatur-üblichen Detektionsmethoden an (z. B. Petrie III und Korytnyk, Anal. Biochem., Vol. 131, p. 153-159, 1983).

In der Regel ergeben sich nach einer Kieselgel-Säulenchromatographie zuckernukleotidhaltige Fraktionen mit unterschiedlicher Reinheit, die je nach Anteil an Verunreinigungen mit Hilfe einer Anionenaustausch-Chromatographie weiter gereinigt, oder aber lediglich mit Hilfe einer Gelfiltration entsalzt werden müssen. Alle das gewünschte Produkt enthaltenden (positiven) Fraktionen werden je nach ihrem Anteil an Verunreinigungen vereinigt und unter Vakuum eingeengt.

Diejenigen Fraktionen, die weiter mit einer Anionenaustausch-Chromatographie gereinigt werden müssen, werden in einem geeigneten Laufmittel gelöst, insbesondere in dem Laufpuffer der Säule. Die weitere Chromatographie verläuft nach dem vom Hersteller angegebenen Elutionspuffer, je nach Art des verwendeten Anionenaustauscher-Materials. Positive Fraktionen werden mit den o. a. Detektionsverfahren detektiert, im Vakuum eingeengt und mit Hilfe einer Gelfiltration weiter entsalzt und gereinigt.

Fraktionen, die nicht mit einer Anionenaustausch-Chromatographie gereinigt werden müssen, können direkt mit Hilfe einer Gelfiltration (z. B. Biogel P2, Sephadex G 10 bis G 200) weiter gereinigt bzw. entsalzt werden. Das Produkt eluiert dabei als das Triethylammonium-Hydrogencarbonat-Salz. Positive Fraktionen werden mit den angegebenen Detektionsverfahren ermittelt, im Vakuum eingeengt und bei -20°C gelagert.

Die so gereinigten Zuckernukleotide zeigen in der NMR-Spektroskopie identische Signale im Vergleich mit Literatur-Spektren.

Die Produkte zeigen sich in biologischen Tests, d. h. bei der Übertragung des Zuckers mit Hilfe einer entsprechenden Glycosyltransferase, aktiv.

Die folgenden Beispiele dienen zur Illustration der Erfindung und sollen diese in keiner Weise einschränken.

### Beispiel 1

Das erfindungsgemäße Verfahren zur Isolierung und Reinigung von nukleotidaktivierten Zuckern soll am Beispiel der Isolierung von Cytidin-Monophosphat-N-Acetyl-Neuraminsäure (CMP-Nana) aus E. coli Z3626 näher erläutert werden. Dieser Bakterienstamm ist literaturbekannt (Steenbergen et al., J. Bacteriol., Vol. 174, p. 1099). Der verwendete E. coli-Stamm besitzt eine Mutation in der Sialyltransferase, die das intrazellulär entstandene CMP-Nana polymerisiert. Durch diesen Defekt häuft sich intrazellulär CMP-Nana an.

### a) Anzucht und Fermentation des Stammes E. coli Z3626

Der Stamm wächst in einem in der Literatur (Uchida et al., Agr. Biol. Chem., Vol. 37, Nr. 9, p. 2105-2110, 1973) beschriebenem Medium. Dieses wurde in seiner Zusammensetzung auf eine hohe intrazelluläre Konzentration von CMP-Nana hin weiter optimiert.

Optimierung der C-Quelle:
Es wurden verschiedene C-Quellen verwendet (Laktose, Saccharose, Maltose, Glukose, Galaktose, Sorbit, Mannose, Glycerin). Dabei erwies sich Glukose als das am besten geeignete Substrat.

Optimierung der Glukose-Konzentration:
Für die Bildung von CMP-Nana erwies sich 30 g/l Glukose als die optimale Konzentration.

Optimierung der N-Quelle:
Es wurden für die Optmimierung sowohl komplexe als auch definierte N-Quellen verwendet. Dabei zeigte sich mit Hefeextrakt die beste Ausbeute.

Optimierung der Hefeextrakt-Konzentration:
Für die Bildung von CMP-Nana erwies sich 2 g/l als die optimale Konzentration.

Optimierung der Kultivierung:
Der Stamm zeigt die höchsten intrazellulären CMP-Nana-Konzentrationen bei einer Kultivierungstemperatur von 30 bis 40°C und einer Kultivierungsdauer von 16 bis 24 Stunden bei guter Belüftung und einem pH-Wert von 6 bis 8.

Intrazelluläre CMP-Nana-Konzentration unter den optimierten Bedingungen: Es werden mit dem Stamm etwa 150mg CMP-Nana/10l Kultur gebildet.

### Beispiel 2

### Gewinnung und Aufbruch der Zellen

Sobald die Zelldichte einen ausreichenden Wert erreicht hat, d. h. nach etwa 16 bis 30 Stunden, wird die Kultivierung abgebrochen. Die Zellen werden durch Zentrifugation geerntet und mit einer Pufferlösung wie etwa Tris/HCl 10 bis 100 mM, pH 6 bis 8 gewaschen. Die sedimentierten Zellen werden in einem geeigneten Puffer (s.o. und unter Zusatz von EDTA 1 bis 10 mM und NaF 0,1 bis 1 mM) resuspendiert und in üblicher Weise, z. B. durch Schütteln mit feinen Glasperlen, Ultraschallbehandlung oder French press, in der Kälte aufgeschlossen. Erleichternd kann dazu noch Lysozym (1 bis 10 mg/ml) verwendet werden. Diese Mischung wird als CMP-Nana Quelle (Rohextrakt) verwendet.
Durch die anschließende Zugabe von Ethanol bis zu einer Endkonzentration von 50 % kommt es zur Fällung der Proteine. Nach Inkubation bei 4°C für 1 Stunde wird zentrifugiert und der Überstand im Vakuum eingeengt (einrotiert bzw. lyophilisiert).

### Beispiel 3

### Reinigung von CMP-Nana mit Hilfe einer Kieselgel-Säulenchromatographie

Der so zur Trockne eingeengte Überstand wird in etwas Isopropanol:1M Triethylammonium-Hydrogencarbonat im Volumenverhältnis 1:1,25 (Laufmittel der Kieselgel-Säule) gelöst und mit trockenem Kieselgel vermischt, so daß eine Masse von honigähnlicher Konsistenz entsteht. Diese Mischung wird auf eine Kieselgel-Säule aufgetragen, mit dem Laufmittel unter Druck eluiert und fraktioniert aufgefangen. Die Verwendung von Triethylammonium-Hydrogencarbonat-Puffer ist wichtig, da so das Produkt in seiner stabilsten Salz-Form gewonnen werden kann.
Die Fraktionen werden mittels geeigneter Detektionsverfahren untersucht, bevorzugt mit Hilfe der Dünnschicht-Chromatographie (DC) oder mit Hilfe der HPLC. Die DC kann auf einem geeigneten Träger (z. B. Kieselgel 60 HPTLC-Platten (Merck) o. ä.) durchgeführt werden. Dabei bietet sich ein Laufmittel mit der Zusammensetzung Isopropanol:1M Ammonium-Acetat 2,4:2 an.
Für die Analytik mit Hilfe der HPLC bieten sich die Literatur-übliche Meßmethoden an (z. B. Petrie III und Korytnyk, Anal. Biochem., Vol. 131, p. 153-159, 1983).

In der Regel ergeben sich nach der Kieselgel-Säulenchromatographie unterschiedliche Reinheitsstufen, die je nach Anteil der Verunreinigung mit Hilfe einer Anionenaustauscher-Chromatographie weiter gereinigt, oder aber lediglich mit Hilfe einer Gelfiltration (z. B. Biogel P2 200 bis 400 mesh (Biorad) oder Sephadex G-10 (Pharmacia)) entsalzt werden müssen.
Alle positiven Fraktionen werden je nach ihrem Anteil an Verunreinigungen vereinigt und unter Vakuum eingeengt.
Diejenigen Fraktionen, die weiter mit einer Anionenaustausch-Chromatographie gereinigt werden müssen, werden in dem Laufmittel der Anionenaustausch-Chromatographie gelöst und auf die Säule aufgetragen.

Bevorzugt wird dazu eine Anionenaustausch-Chromatographie mit Dowexdurchgeführt.
Fraktionen, die nicht über eine Anionenaustausch-Chromatographie gereinigt werden müssen, können mit Hilfe einer Gelfiltration (bevorzugt Biogel P2) weiter gereinigt bzw. entsalzt werden. Das Produkt eluiert als Triethylammoniumhydrogencarbonat-Salz.
Positive Fraktionen werden mit den oben angegebenen Detektionsverfahren ermittelt, lyophilisiert und bei -20°C eingefroren.

Die so gereinigten Proben wurden mit Hilfe der ¹H-NMR-Spektroskopie auf die richtige Struktur und eventuelle, mit den erwähnten Meßmethoden nicht erfaßbare Verunreinigungen hin untersucht und mit Literaturspektren verglichen.

### Beispiel 4

### Untersuchung des Produktes mit Hilfe der ¹H-NMR-Spektroskopie (Liu et al., J. Am. Chem. Soc., Vol. 114, p. 3901, 1992)

¹H-NMR: (300 MHz, D₂O):
δ 1.66(1 H, ddd, J = 13, 12 Hz, H-3ax), 2.05 (3 H, s, NAc), 2.5 (1 H, dd, J = 13, 4.8 Hz, H-3eq), 3.46 (1 H, d, J = 9.6 Hz, H-7), 3.63 (1 H, dd, J = 6.6, 12 Hz, H-9a), 3.9 (1 H, dd, J = 12, 2.4 Hz, H-9b), 3.94 bis 4.0 (2 H, m, H-8, H-5) 4.06 (1 H, ddd, J = 10,5, 12 Hz, H-5), 4.15(1 H, dd, J = 10, 1.5 Hz, H-6), 4.22-4.28 (3 H, m, H-4', H-5'), 4.29-4.38 (2 H, m, H-3', H-2') 6.00 (1 H, d, J = 5 Hz, H-1'), 6.13 (1 H, d, J = 7.8 Hz, H-5"), 7.98 (1 H, d, J = 7.6 Hz, H-6")

### Beispiel 5

### Enzymatische Synthese von α-D-Neu5Ac-(2-6)-β-D-Gal-(1-4)-β-D-GlcNAc-O(CH₂)₆NH₂

Das gebildete Produkt ist von der Reinheit her für enzymatische Umsetzungen (Sialyltransferase-Reaktion) einsetzbar und aktiv.

12 mg (25 µmol) β-D-Gal-(1-4)-β-D-GlcNAc-O(CH₂)₆NH₂ werden in 2 ml 0.05M Cacodylat-Puffer gelöst und mit 15.4 mg (25 µmol) nach Beispiel 2 hergestellter CMP-Neuraminsäure, 1.5 mg Rinderserumalbumin und 2 mg MnCl₂ versetzt. Nach Einstellen des pH auf 7.4 werden 40 mU α-2-6-Sialyltransferase (aus Rattenleber, Boehringer Mannheim) und 20 U alkalische Phosphatase (aus Kälberdarm, Boehringer Mannheim) zugegeben und 10 Tage bei Raumtemperatur inkubiert. Zur Aufarbeitung wird über Biogel P2 (Biorad) mit Wasser als Elutionsmittel chromatographiert. Nach Gefriertrocknung erhält man das Trisaccharid. Ausbeute: 11 mg
α-D-Neu5Ac-(2-6)-β-D-Gal-(1-4)-β-D-GlcNAc-O(CH₂)₆NH₂.

¹H-NMR (300 MHz, D₂O):
δ 1.35 bis 1.42 (4 H, m, CH2-Spacer), 1.54 bis 1.7 (4 H, m, CH2-Spacer); 1.72 (1 H, dd, H-3ax), 2.04 (3 H, s, NAc_{Neu5Ac}), 2.06 (3 H, s, NAc_{GlcNAc}), 2.68 (1 H, dd, H-3eq), 3.0 (2 H, t, CH₂-Spacer), 3.5 bis 4.0 (21 H, m), 4.46 (1 H, d, H-1-Gal), 4.56 (1 H, d, H-1-GlcNAc)

¹³C-NMR (300 MHz, D₂O):
δ 22.35 (CH₃-Neu5Ac), 22.63 (CH₃-GlcNAc), 24.93, 25.54, 26.97, 28.65 (Spacer-CH₂), 39.72 (CH₂-NH₂), 40.40 (C3"), 52.18 (C5 "), 55.20 (C2), 60.71 (C6), 62.97 (C9"), 63.67 (C6'), 68.53 (C7"), 68.72 (C4', C4"), 70.64 (CH₂-O), 71.03 (C2'), 72.0 (C8"), 72.77 (C3, C3'), 72.85 (C6 "), 74.0 (C5'), 74.78 (C5), 81.1 (C4), 100.8 (C2"), 101 (C1), 104 (C1') 173.5 (C1"), 174.5 (Ac-GlcNAc), 175 (Ac-Neu5Ac)

## Patentansprüche

1. Verfahren zur Isolierung und Reinigung von Zuckernukleotiden aus biologischen Quellen, bei welchem eine notwendigenfalls von unlöslichen Zellbestandteilen befreite, zuckernukleotidhaltige Lösung, die ein Zellextrakt oder eine Reaktionslösung eines enzymatischen Reaktionsansatzes sein kann, nach Entfernung gelöster Proteine mittels alkoholischer Proteinfällung bis zur Trockne eingeengt, der resultierende, zuckernukleotidhaltige Rückstand in einem Laufmittelgemisch aufgenommen und an Kieselgel chromatographiert wird, dadurch gekennzeichnet, daß der zuckernukleotidhaltige Rückstand in einem aus einer Mischung eines kurzkettigen Alkohols mit einer 0,5 bis 1 molaren wäßrigen Lösung eines Ammoniumsalzes in einem Volumenverhältnis von 1:1 bis 1:10 bestehenden Laufmittelgemisch aufgenommen, mit trockenem Kieselgel vermengt als zähe Masse von honigartiger Konsistenz auf eine chromatographische Säule mit handelsüblichem Kieselgel beliebiger Korngröße als stationärer Phase aufgetragen und mit dem genannten Laufmittelgemisch unter Druck eluiert wird.

2. Verfahren nach Anspruch 1, dadurch gekennzeichnet, daß das Laufmittelgemisch aus einer Mischung eines kurzkettigen Alkohols mit einer 0,5 bis 1 molaren wäßrigen Lösung eines Ammoniumsalzes in einem Volumenverhältnis von 1:1 bis 1:2 besteht.

3. Verfahren nach Anspruch 1 oder 2, dadurch gekennzeichnet, daß das Volumenverhältnis 1:1,25 beträgt.

4. Verfahren nach einem der Ansprüche 1 bis 3, dadurch gekennzeichnet, daß die wäßrige Lösung des Ammoniumsalzes 1 molar ist.

5. Verfahren nach einem der Ansprüche 1 bis 4, dadurch gekennzeichnet, daß der kurzkettige Alkohol 2-Propanol ist.

6. Verfahren nach einem der Ansprüche 1 bis 5, dadurch gekennzeichnet, daß das Ammoniumsalz Triethylammoniumhydrogencarbonat ist.

7. Verfahren nach einem der Ansprüche 1 bis 6, dadurch gekennzeichnet, daß das Zuckernukleotid Cytidin-Monophosphat-N-Acetyl-Neuraminsäure ist.

## Claims

1. A process for isolating and purifying sugar nucleotides from biological sources, in which process a sugar nucleotide-containing solution, which, if necessary, has been freed from insoluble cell constituents, and which can be a cell extract or a reaction solution from an enzymic reaction mixture, is concentrated to dryness, after removing dissolved proteins by means of alcoholic protein precipitation, and the resulting, sugar nucleotide-containing residue is taken up in an eluent mixture and chromatographed on silica gel, which comprises taking up the sugar nucleotide-containing residue in an eluent mixture composed of a mixture of a short-chain alcohol and a 0.5 to 1 molar aqueous solution of an ammonium salt in a ratio by volume of 1:1 to 1:10, mixing the resulting solution with dry silica gel, loading it, as a viscous mass of honey-like consistency, onto a chromatography column containing commercially available silica gel of arbitrary particle size as the stationary phase, and eluting the sugar nucleotide under pressure using the said eluent mixture.

2. The process as claimed in claim 1, wherein the eluent mixture is composed of a mixture of a short-chain alcohol and a 0.5 to 1 molar aqueous solution of an ammonium salt in a ratio by volume of 1:1 to 1:2.

3. The process as claimed in claim 1 or 2, wherein the ratio by volume is 1:1.25.

4. The process as claimed in one of claims 1 to 3, wherein the aqueous solution of the ammonium salt is 1 molar.

5. The process as claimed in one of claims 1 to 4, wherein the short-chain alcohol is 2-propanol.

6. The process as claimed in one of claims 1 to 5, wherein the ammonium salt is triethylammonium hydrogen carbonate.

7. The process as claimed in one of claims 1 to 6, wherein the sugar nucleotide is cytidine monophosphate-N-acetylneuraminic acid.

## Revendications

1. Procédé pour l'isolement et la purification de nucléotides-oses à partir de sources biologiques, dans lequel, une solution contenant de nucléotides-oses, si nécessaire, débarrassée de constituants cellulaires insolubles, ladite solution peut être un extrait cellulaire ou une solution réactionnelle d'une charge enzymatique, après élimination des protéines dissoutes au moyen de précipitation de protéines à l'alcool, on évapore jusqu'à siccité, on reprend dans un mélange d'éluants le résidu résultant contenant les nucléotides-oses et on chromatographie sur gel de silice, caractérisé en ce qu'on reprend le résidu contenant les nucléotides-oses dans un mélange d'éluants constitué d'un alcool à chaîne courte et d'une solution aqueuse d'un sel d'ammonium de 0,5 à 1M, dans un rapport volumique de 1:1 à 1:10, on mélange avec du gel de silice sec, on porte sous forme de masse de consistance mielleuse sur une colonne de chromatographie garnie de gel de silice avec une granulométrie quelconque en tant que phase stationnaire, et on élue sous pression avec le mélange d'éluants cité.

2. Procédé selon la revendication 1, caractérisé en ce que le mélange d'éluants consiste en un mélange d'un alcool à chaîne courte avec une solution aqueuse de 0,5 à 1M d'un sel d'ammonium dans un rapport volumique de 1:1 à 1:2.

3. Procédé selon la revendication 1 ou 2, caractérisé en ce que le rapport volumique s'élève à 1:1,25.

4. Procédé selon une des revendications 1 à 3, caractérisé en ce que la solution aqueuse du sel d'ammonium présente une concentration 1M.

5. Procédé selon une des revendications 1 à 4, caractérisé en ce que l'alcool à chaîne courte est le 2-propanol.

6. Procédé selon une des revendications 1 à 5, caractérisé en ce que le sel d'ammonium est le bicarbonate de triéthylammonium.

7. Procédé selon une des revendications 1 à 6, caractérisé en ce que le nucléotide-ose est l'acide cytidine-monophosphate N-acétyl-neuraminique.
